# EUROPEAN PATENT APPLICATION

(11) **EP 4 273 669 A1**
(43) Date of publication of application: **08.11.2023**
(21) Application number: 22172094.9
(22) Date of filing: 06.05.2022
(51) Int. Cl.: G06F 3/01, H04M 1/60, A61B 5/16, G06V 40/16

(54) **MONITORING OF FACIAL CHARACTERISTICS**

(71) Applicant: Nokia Technologies Oy, 02610 Espoo (FI)
(72) Inventor: Lehtiniemi, Arto, 33100 Tampere (FI); Salmimaa, Marja, 33100 Tampere (FI)
(74) Representative: Swindell & Pearson Limited

(57) **Abstract**

Examples of the disclosure relate to monitoring of facial characteristics. In examples of the disclosure there may be provided an apparatus that is configured to determine that a communications device is positioned close to an ear of a user of the communication device during a communication session wherein the communications device comprises at least one display. The apparatus may also be configured to use sensors in or under the display of the communications device to monitor one or more facial characteristics of the user while the communication device is close to the user's ear and to identifying an emotional context of the user based on the monitored one or more facial characteristics of the user.

## Description

### TECHNOLOGICAL FIELD

Examples of the disclosure relate to monitoring of facial characteristics. Some relate to monitoring of facial characteristics so as to enable emotional contexts of a user to be identified.

### BACKGROUND

Monitoring facial characteristics of a user can be useful as it can give an indication of the emotional context of the user and/or any other suitable information.

### BRIEF SUMMARY

According to various, but not necessarily all, examples of the disclosure there may be provided apparatus comprising means for:
determining that a communications device is positioned close to an ear of a user of the communication device during a communication session wherein the communications device comprises at least one display;
using sensors in or under the display of the communications device to monitor one or more facial characteristics of the user while the communication device is close to the user's ear; and
identifying an emotional context of the user based on the monitored one or more facial characteristics of the user.

The means may be for enabling information indicative of the identified emotional context to be transmitted to one or more other participants in the communication session.

The means may be for detecting a user input indicating whether or not the user of the communications device wants to transmit information indicative of the identified emotional context to one or more other participants in the communication session and determining whether or not to transmit the information indicative of the identified emotional context to the one or more other participants in the communication session based on the user input.

The information indicative of the identified emotional context may be configured to enable a visual representation of the identified emotional context to be generated.

The visual representation may comprise one or more of; an avatar, an animated emoji, an image of the user of the communications device.

The communication session may comprise a video call and the means are for using the visual representation of the identified emotional context to replace images from the imaging device of the communication device in the video call.

The sensors in or under the display of the communications device may comprise optical sensors.

The one or more facial characteristics may comprise one or more of: the position of one or more facial features, relative distances between two or more facial features, movement of one or more facial features, skin tone.

The means may be for controlling the communications device to provide an output to the user to indicate the identified emotional context.

The communications device may comprise a tactile output device and the means may be for controlling the tactile output device to provide the output indicating the identified emotional context.

The means may be for detecting a user input indicating whether or not the identified emotional context is correct.

The communications device comprises one or more microphones and the means are for analysing microphone output signals to determine when the user is speaking and controlling the monitoring of the one or more facial characteristics to monitor the one or more facial characteristics of the user while they are speaking.

The means may be for identifying the emotional context using a machine learning program.

According to various, but not necessarily all, examples of the disclosure, there may be provided a device comprising an apparatus as described herein wherein the device is at least one of: a telephone, a camera, a computing device, a teleconferencing device, a virtual reality device, an augmented reality device.

According to various, but not necessarily all, examples of the disclosure, there may be provided a method comprising:
determining that a communications device is positioned close to an ear of a user of the communication device during a communication session wherein the communications device comprises at least one display;
using sensors in or under the display of the communications device to monitor one or more facial characteristics of the user while the communication device is held to the ear; and
identifying an emotional context of the user based on the monitored one or more facial characteristics of the user.

According to various, but not necessarily all, examples of the disclosure, there may be provided a computer program comprising computer program instructions that, when executed by processing circuitry, cause:
determining that a communications device is positioned close to an ear of a user of the communication device during a communication session wherein the communications device comprises at least one display;
using sensors in or under the display of the communications device to monitor one or more facial characteristics of the user while the communication device is held to the ear; and
identifying an emotional context of the user based on the monitored one or more facial characteristics of the user.

### BRIEF DESCRIPTION

Some examples will now be described with reference to the accompanying drawings in which:
FIG. 1 shows an example communications device;
FIG. 2 shows an example method;
FIGS. 3A and 3B show an example communications device in use;
FIGS. 4A and 4B show an example communications device in use;
FIGS. 5A and 5B show an example communications device in use;
FIGS. 6A and 6B show an example communications device in use;
FIGS. 7A and 7B show an example communications device in use; and
FIG. 8 shows an example apparatus.

### DETAILED DESCRIPTION

If a user is participating in a video communication session then other participants in the session can view images of the user. This can enable the other participants to see the facial expressions of the user. This facial expression allows the other participants to determine the emotional context of the user. For example, the other participants can see if the user is happy or sad or experiencing any other type of emotion. However, if the devices used for the video telecommunication session are not able to obtain images of the user's face then this information cannot be provided to the other participants in the communication session. Examples of the disclosure enable information about a user's emotional context to be obtained even if the devices used for the communication session are not able to obtain images of the user's face.

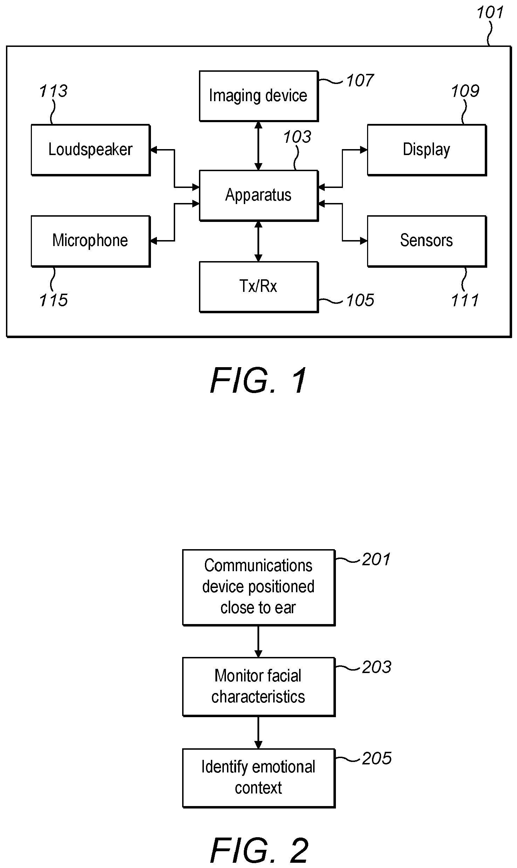

Fig. 1 schematically shows an example communications device 101 that could be used to implement examples of the disclosure. The communications device 101 could be a telecommunications device 101 such as a mobile phone, a camera, a computing device, a teleconferencing device, a virtual reality device, an augmented reality device or any other suitable type of device.

The example communications device 101 comprises an apparatus 103, a transmitter/receiver 105, an imaging device 107, a display 109, one or more sensors 111, a loudspeaker 113 and a microphone 115. Only components that are referred to in the following description are shown in Fig.1. Additional components could be provided in some examples of the disclosure. For instance, the communications device 101 could comprise a power source, a tactile output device or any other suitable components.

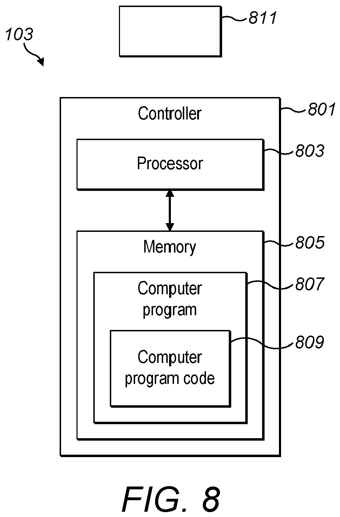

The apparatus 103 can comprise a controller comprising a processor and memory. Examples of an apparatus 103 are shown in Fig. 8. The apparatus 103 can be configured to enable control of the communications device 101. For example, the apparatus 103 can be configured to control the images that are displayed on the display 109 and/or the images that are captured by the imaging device 107.

The transmitter/receiver 105 can comprise any means that enables the communications device 101 to participate in communication sessions. The transmitter/receiver 105 can enable the communications device 101 to communicate in communications networks. The communications networks can be wired and/or wireless communications networks.

The imaging device 107 can comprise any means for capturing an image. The imaging device 107 can comprise one or more cameras or any other suitable means for capturing images. In the example of Fig.1 only one imaging device 107 is shown. However, it is to be appreciated that the communications device 101 could comprise a plurality of imaging devices 107 that could be positioned in different locations within the communications device 101.

The imaging device 107 can comprise one or more sensors where the sensors can be configured to detect images. The sensors of the imaging device 107 can comprise any suitable type of image sensor. For instance, the sensor of the imaging device 107 can comprise a digital image sensor such as a charge-coupled-device (CCD) or a complementary metal-oxide-semiconductor (CMOS).

The imaging device 107 can be controlled by the apparatus 105 to enable images to be captured. Once an image has been captured it can be stored in a memory and/or transmitted to a third party.

The display 109 can comprise any means that can be configured to enable information to be displayed. The display can enable visual rendering of images or other information. The display 109 can be controlled by the apparatus 103 to display images captured by the imaging device 107 and/or images received from the communications network and/or any other suitable type of images.

The sensors 111 can comprise any means that can be configured to detect facial characteristics of a user of the communications device 101. The one or more sensors 111 can be embedded within the display 109 or in any other suitable location within the device 111.

The sensors 111 can comprise any suitable types of sensor 11 that can be configured to detect information that is indicative of a facial characteristic of a user. In some examples the sensors 111 could comprise optical sensors, the optical sensors could be configured detect different wavelengths of light to the sensors within the imaging device 107. For instance, the optical sensors could be configured to detect infrared light. The light that is detected by the optical sensors can be generated and emitted from the device 101. For example, the device 101 could comprise one or more light emitting diodes or other light sources configured to emit light at an appropriate wavelength. Other types of sensors 111 such as capacitive sensors, ultrasound sensors, mm wave sensors could be used in other examples of the disclosure. Where the sensors 111 are configured to detect a reflected signal the device 101 can be configured to transmit an original signal. For instance, if the sensors 111 are ultrasound sensors or mm wave sensors then the device 101 can comprise one or more emitters configured to emit ultrasound signals or mm wave signals.

The sensors 111 could be configured to perform different functions during different use scenarios of the communications device 101. For instance, in examples of the disclosure the sensors 111 can be configured to detect facial characteristics of the user's face. In other scenarios the same sensors 111 could be used to detect a user's fingerprint or any other suitable information.

The loudspeaker 113 can comprise any means for providing an audio output to a user of the communications device 101. The loudspeaker 113 can comprise any means for converting an electrical input signal to a sound signal. The loudspeaker 113 could be embedded within the communications device 101 or could be part of a peripheral device such as a headset or earbud. The loudspeaker 113 can enable audio from a communication session to be played back for the user of the communications device 101.

The microphone 115 can comprise any means for capturing an audio input from a user of the communications device 101. The microphone 115 could be embedded within the communications device 101 or could be part of a peripheral device such as a headset. The microphone 115 can comprise any means that can be configured to convert a sound signal into an electrical microphone signal. The electrical microphone signal can be used for communication sessions to enable the captured sounds to be transmitted to other devices within the communication session.

Fig. 2 shows an example method according to examples of the disclosure. The method could be implemented using an apparatus 103 and communications device 101 as described above or using any other suitable type of communications device 101 and/or apparatus 103 that comprises a display 109.

The method comprises, at block 201, determining that a communications device 101 is positioned close to an ear of a user of the communication device 101 during a communication session.

The communication session could comprise a communication session with one or more other communications devices. In some examples the communication session could comprise a video call or any other session in which both images and audio are transferred between the communications device 101 and the other participant devices in the communication session. The communication session can enable the participants within the communication session to view images of the other participants as they talk to teach other. For example, the communications device 101 can capture images using the imaging device 107 at the same time as the sound is captured using a microphone 115 and can enable the images and corresponding microphone signals to be transmitted to the other participants within the communication session.

Any suitable means can be used to determine that the communications device 101 is positioned close to the user's ear. For example, the one or more sensors 111 of the communications device 101 could comprise one or more proximity sensors that could detect how close the communications device 101 is to the user's head. Other types of sensors could be used instead, or in addition to this, for examples images obtained by the imaging device could show how close the communications device 101 is to the user's head. In some examples, the communications device 101 could also comprise accelerometers or other positioning means that could determine movement and/or a particular orientation of the communications device 101 that indicates that the communications device 101 has been positioned close to the user's ear.

It can be determined that the communications device 101 is close to the user's ear if the communications device 101 is within a threshold distance of the user's ear. For example, if the communications device 101 is within a few cm of the user's ear.

In some examples the communications device 101 could be a hand held device, such as a mobile telephone, and it could be determined that the user is holding the handled device adjacent to, or in proximity, to their ear. For instance, the user could be holding their communications device 101 to a side of their head so that a loudspeaker 113 of the communications device 101 is close to their ear and a microphone 115 of the communications device 101 is close to their mouth.

When the communications device 101 is arranged in a position so that the communications device 101 is close to the user's ear, the display 109 of the communications device 101 is positioned close to the user's face, or part of the user's face.

At block 203 the method comprises using sensors 111 in or under the display 109 of the communications device 101 to monitor one or more facial characteristics of the user while the communication device 101 is close to the user's ear.

The facial characteristics can comprise the position of one or more facial features, relative distances between two or more facial features, movement of one or more facial features, skin tone or any other suitable features. The facial features can comprise features that change as a user's facial expression changes. A user's facial expression can change by the user moving muscles within their face. For example, a user can smile or frown which would cause movement of the muscles around the mouth and/or eyes. This movement of the muscles would also cause changes in the position of facial features on the user's skin. For example, it could change the appearance of fine lines and wrinkles and/or it could change the positions of freckles or other marks on the user's face. The sensors 111 can be configured to detect such facial characteristics. In some examples the sensors 111 could be configured to detect changes in the facial characteristics, for example it could detect movement of fine lines or markings. In some examples the sensors 111 could be configured to detect particular configurations of the facial characteristics. For example, a particular configuration of the fine lines or markings could be known to correspond to a particular facial expression or emotional context.

Other types of facial characteristics could be detected in other examples of the disclosure. For instance, the user's skin tone could be detected. This could give an indication of whether or not a user is blushing or flushed. This can give more information about a user's emotional context.

At block 205 the method comprises identifying an emotional context of the user based on the monitored one or more facial characteristics of the user. The emotional context can be an indication of the current emotions of the user. This could be whether the user is happy, sad, laughing, angry or in any other suitable state.

In some examples the emotional context could comprise different levels of the same emotions. For example, a user could be identified as being very happy or just a bit happy.

In some examples the communications device 101 can be configured to automatically determine the emotional context of the user. This can enable the emotional context to be determined without any specific input from the user. In other examples the communications device 101 can be configured to enable a user to make an input to confirm that an emotional context has been identified correctly. For instance, the communications device 101 can be configured to provide an output to the user to indicate the identified emotional context. The output could be provided via a tactile output device or any other suitable means. The communications device 101 could then enable a user to make an input indicating whether or not the correct emotional context has been indicated.

Any suitable means can be used to identify the emotional context of the user. In some examples the emotional context can be determined using a machine learning program.

The machine learning program used to identify the emotional context of the user can comprise a neural network or any other suitable type of trainable model. The term "machine learning program" refers to any kind of artificial intelligence (Al), intelligent or other method that is trainable or tuneable using data. The machine learning program can comprise a computer program. The machine learning program can be trained or configured to perform a task, such as identifying an emotional context of a user based on detected facial characteristics, without being explicitly programmed to perform that task or starting from an initial configuration. The machine learning program can be configured to learn from experience E with respect to some class of tasks T and performance measure P if its performance at tasks in T, as measured by P, improves with experience E. In these examples the machine learning program can learn from previous outputs that were obtained for the same or similar inputs. The machine learning program can also be a trainable computer program. Other types of machine learning models could be used in other examples.

Any suitable process can be used to train or to configure the machine learning program. The training or configuration of the machine learning program can be performed using real world or simulation data. The training of the machine learning program can be repeated as appropriate until the machine learning program has attained a sufficient level of stability. The machine learning program has a sufficient level of stability when fluctuations in the outputs provided by the machine learning program are low enough to enable the machine learning program to be used to identify the emotional context of a user. The machine learning program has a sufficient level of stability when fluctuations in the predictions provided by the machine learning program are low enough so that the machine learning program provides consistent responses to test inputs.

In some examples the training of the machine learning program can be repeated as appropriate until the machine learning program provides consistent responses to test inputs and/or until a predefined accuracy has been attained and/or until any other suitable criteria are satisfied.

Once the emotional context has been determined this information can be used for any suitable purpose. In some examples information indicative of the identified emotional context can be transmitted to one or more other participants in the communication session.

In some examples the communications device 101 can be configured to enable a user to control whether or not the information indicative of the emotional context is shared with the other participants within the voice call. For instance, in some examples the user could make an input indicating whether or not they want the information indicative of the emotional context to be shared. The communications device 101 can then be configured to determine whether or not to transmit the information indicative of the identified emotional context to the one or more other participants in the communication session based on the user input.

The information indicative of the identified emotional context can be configured to enable a visual representation of the identified emotional context to be generated. This can enable the other participant devices within the communication session to generate images or other visual representations corresponding to the identified emotional context. The visual representation can comprise one or more of; an avatar, an animated emoji, an image of the user of the communications device 101 and/or any other suitable type of visual representation.

In examples where the communication session comprises a video call the visual representations of the identified emotional context can replace images from the imaging device 107 of the communication device 101 in the video call. For instance, the imaging device 107 could not be able to capture images of the user's face because the communications device 101 is positioned adjacent to the user's head. Therefore, the images of the user's face can be replaced with the visual representations of the identified emotional context. The process of generating the visual representations and/or the process of replacing images of the user's face with the visual representations can be performed by the device 101, by a server within a communications network, by the participant's device or by any other suitable device or combination of devices.

The communications device 101 could be configured to enable other uses for the information indicative of the identified emotional context. For example, this information could be used to track the emotional state of a user for health and wellbeing purposes or could be used to control other applications of the communications device 101.

In some examples the communications device 101 is configured so that the user's facial characteristics are only monitored at particular times. For instance, the communications device 101 might be configured so that the user's facial characteristics are only monitored when the user is speaking. In such examples the microphone signals obtained from the one or more microphones 115 of the communications device 101 can be monitored to determine when the user is speaking.

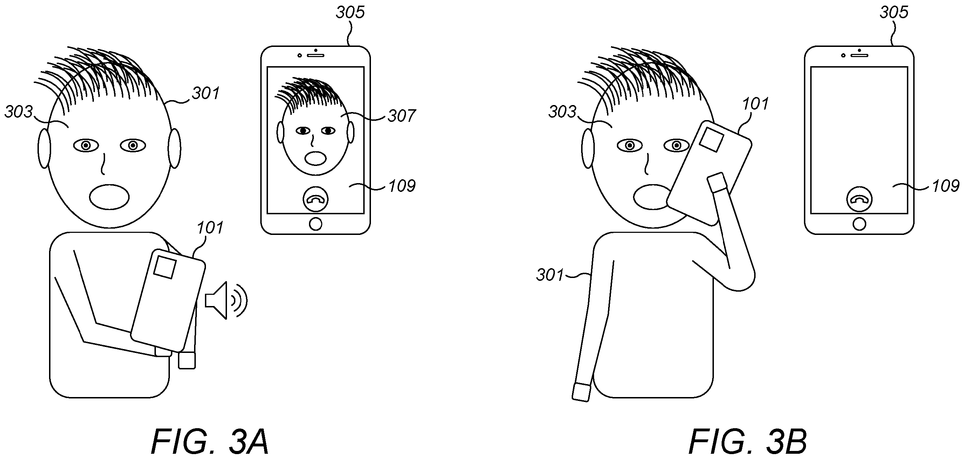

Figs. 3A and 3B show an example communications device 101 in use. The communications device 101 could be a communications device 101 as shown in Fig. 1 or any other suitable type of device. In this example the communications device 101 is a handheld device such as a mobile phone. Other types of device could be used in other examples

In Fig. 3A the user 301 of the communications device 101 is participating in a communication session. In this example the communication session is a video call. In Fig. 3A the user 301 is holding the communications device 101 in front of their face 303 and at a distance that enables the imaging device 107 to obtain images of the user's face 303.

The images 307 of the user's face 303 can then be transmitted to other participant devices 305 within the communication session. The images 307 of the user's face 303 can be displayed on the display 109 of the participant devices 305. This enables the other participants in the communication session to see the images 307 of the user's face. This can enable non-verbal communication during the voice call. For example, a user 301 smiling or frowning conveys information about their emotional context.

When the user 301 is using the communications device 101 in this way the audio from the communication session is played back through the loudspeaker 113 of the communications device 101. This means that the audio is not private and could be heard by other people around the user 301.

If the user 301 wishes to make the communication session more private they can change the way they are holding the communications device 101 and hold it close to their ear as shown in Fig. 3B.

When the communications device 101 is held close to the user's ear the imaging device 107 can no longer capture images of the user's face 303. This means that the images 307 of the user's face are no longer transmitted to other participant devices 305 within the communication session. The other participant devices 305 now display a blank screen instead of the images 307 of the user's face. This means that the users of the participant devices are not getting any additional information about the user's emotional context. For example, they don't know if the user is smiling or frowning.

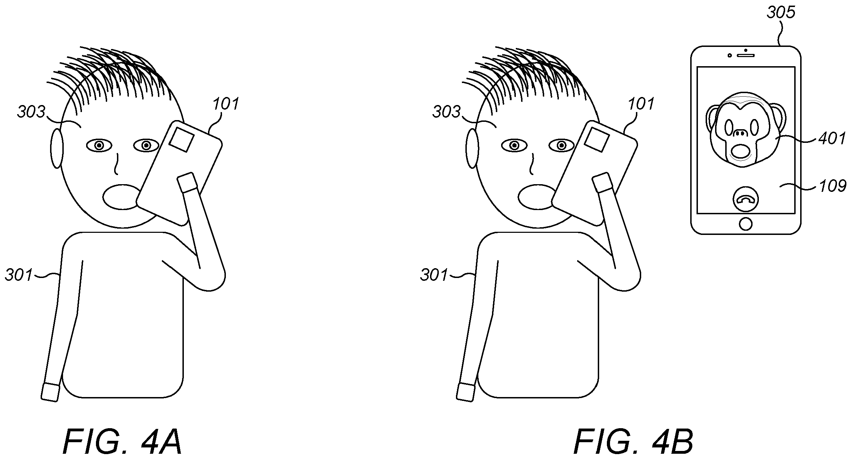

Figs. 4A and 4B show another example scenario for a communications device 101 in use. The communications device 101 could be a communications device 101 as shown in Fig. 1 or any other suitable type of device. In this example the communications device 101 is a handheld device such as a mobile phone. Other types of communications device 101 could be used in other examples.

In Fig. 4A the user is 301 is using the communications device 101 for a voice call or other similar communication session. The user 301 is holding the communications device 101 close to their ear for privacy purposes or for any other suitable reasons. This means that the imaging device 107 of the communications device 101 cannot obtain images for the user's face 303 for use in the communication session.

In examples of the discourse the sensors 111 are configured to monitor facial characteristics of the user's face 303. The sensors 111 can be optical sensors or any other suitable type of sensors. In some examples the sensors 111 could be configured to detect infrared light that is reflected by the user's face 303 so as to obtain information about the facial characteristics.

In some examples the sensors 111 that are used for monitoring the facial characteristics can be embedded within the display 109 of the communications device 101. For instance, the sensors 111 could be integrated within the display 109 so that the sensors 111 are positioned between respective layers or components of the display 109. In some examples the sensors 111 could be positioned underneath the display. The sensors 111 can be positioned within the footprint of the display 109 so that the sensors 111 sense a parameter through at least part of the display 109. The sensors 111 can be located within the device 101 so that they are positioned between a rear surface of the device 101 and the surface of the display 109.

When the communications device 101 is held, or otherwise positioned, close to the user's ear, as shown in Fig. 4A, the display 109 of the communications device 101 is adjacent to and facing the user's face 303. This enables the sensors 111 to be used to detect facial characteristics.

Once the facial characteristics have been detected they can be used to identify the emotional context of the user 301. For instance, a machine learning program can be used to classify the detected facial characteristics as corresponding to a particular emotional context.

Once the emotional context has been identified a visual representation 401 of the identified emotional context may be generated. The visual representation 401 is an image or graphic that can be displayed on the display 109 of the other participant devices 305 in the communication session.

Fig. 4B shows an example visual representation 401. In the example of Fig. 4B the visual representation 401 is an animated emoji. The animated emoji in this case is a monkey, other types of emoji could be used in other examples. The monkey is animated to have facial expression corresponding to the emotional context of the user 301. For instance, if it is determined that the user 301 is happy then the monkey is animated to show happy facial expressions.

In other examples previously obtained images of the user 301 of the device could be used as the visual representation 401. For example, video images or other images showing various facial expressions of the user 301 corresponding to the emotional contexts could be stored in a memory of the communications device 101. When an emotional context of the user 301 is identified a corresponding image can then be retrieved from the memory.

Other types of visual representations 401 could be used in other examples, for instance an avatar or other animation that could be designed to resemble the user 301 could be used.

The visual representation 401 enables the participants using the participant devices 305 to continue to view images corresponding to the user 301 during the communication session. This can reduce disruptions in the communication session due to images from the imaging device 107 no longer being available because the images can be replaced with a different type of image. The visual representations 401 also enable the participants using the participant devices 305 to obtain non-verbal information via the communication session. In particular the visual representations 401 provide an indication of the emotional state of the user 301.

In some examples information could be displayed on the display 109 of the participant device 305 in addition to the visual representation 401. For example, a notification could be displayed that informs the participants using the participant devices 305 that the user 301 has switched to earpiece mode.

In some examples additional information could be provided with the visual representations 401. For instance, in some examples an audio output could be provided with the visual representation 401. The audio output could be mixed in with audio from the communication session. In some examples the audio output could indicate the emotional context of the user 301. In some examples the audio output could indicate which user 301 the visual representation relates to. For instance, if there are more than two participants in the communication session then the audio output can indicate which users are represented by the visual representation.

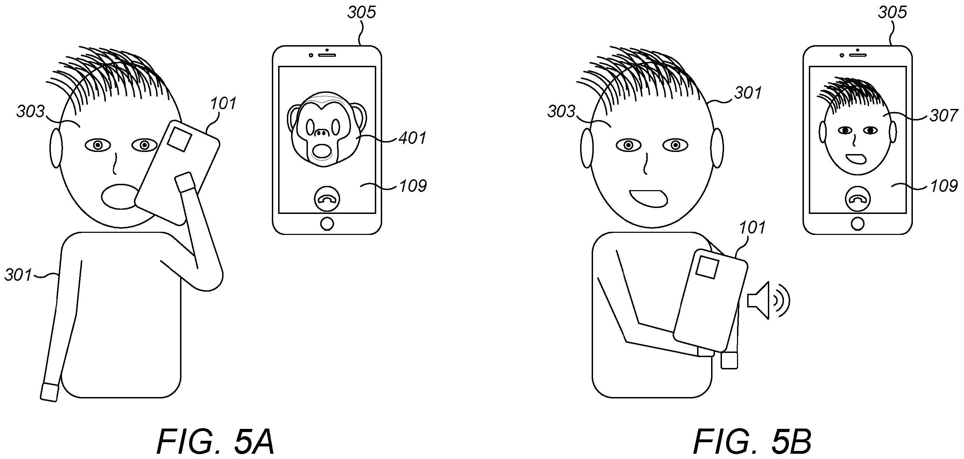

Figs. 5A and 5B show another example scenario for a communications device 101 in use. The communications device 101 could be a communications device 101 as shown in Fig. 1 or any other suitable type of device. In this example the communications device 101 is a handheld device such as a mobile phone. Other types of communications device 101 could be used in other examples.

In Fig. 5A the user is 301 is using the communications device 101 for a voice call or other similar communication session. The user 301 is holding the communications device 101 close to their ear for privacy purposes or for any other suitable reasons. The sensors 111 in the communications device 101 are monitoring the facial characteristics of the user 301 and the outputs of these sensors can enable an emotional context of the user 301 to be identified. A visual representation 401 of the identified emotional context has been generated and is displayed on the display 109 of the other participant devices 305 in the communication session.

In Fig. 5B the user stops holding the communications device 101 close to their ear and instead starts holding the communications device 101 in front of their face 303 and at a distance that enables the imaging device 107 to obtain images of the user's face 303.

The communications device 101 can detect that it is no longer positioned close to the user's ear and/or that the imaging device 107 can now capture images of the user's face 303. For example, the imaging device 107 can detect image data that indicates that the communications device 101 is now held in front of the user's face 303. In some examples positioning sensors could detect the change in position and/or orientation of the communications device 101. Other means for detecting the change in position of the communications device 101 could be used in other examples of the disclosure.

Once it is detected that the communications device 101 can detect that it is no longer positioned close to the user's ear and/or that the imaging device 107 can now capture images of the user's face 303, the images captured by the imaging device 107 can be used in the communication session. The visual representation 401 of the user's emotional context can be replaced with the images 307 of the user's face 303. The images 307 of the user's face 303 can now be displayed on the display 109 of the participant devices 305 in place of the visual representation 401.

This enables the communications device 101 to switch between different modes of operation during the same communication session. Whenever the user 301 changes the position or hold of the communications device 101 the images that are used for the participant devices can be updated. This provides for a seamless and uninterrupted communication session for the other participants. This can also enable the user 301 to continue to provide non-verbal information such as facial expressions even when they change the way they are holding or using the communications device 101.

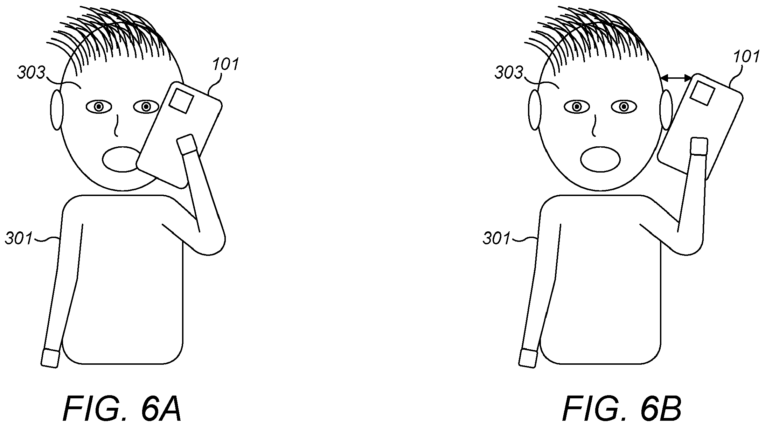

Figs. 6A and 6B show another example scenario for a communications device 101 in use. The communications device 101 could be a communications device 101 as shown in Fig. 1 or any other suitable type of device. In this example the communications device 101 is a handheld device such as a mobile phone. Other types of communications device 101 could be used in other examples.

In Fig. 6A the user is 301 is using the communications device 101 for a voice call or other similar communication session. The user 301 is holding the communications device 101 close to their ear for privacy purposes or for any other suitable reasons. The sensors 111 in the communications device 101 are monitoring the facial characteristics of the user 301 and the outputs of these sensors 111 can be used to identify emotional context of the user 301.

In the example of Fig. 6A, before the information indicative of the identified emotional context is transmitted and/or before the visual representation 401 is generated, the communications device 101 can be configured to provide an opportunity for the user 301 to confirm whether or not the correct emotional context has been identified.

In order to enable the user 101 to confirm whether or not the correct emotional context has been identified the communications device 101 can be configured to provide an output indicative of the identified emotional context. The communications device 101 can be configured to detect a user input in response to the output where the use input can indicate whether or not the correct emotional context has been identified.

In the example of Fig. 6A the output indicative of the identified emotional context can be provided by a tactile output device or by any other suitable means. The tactile output device can provide a tactile or haptic output that a user 301 can perceive through a sense of touch. The use of the tactile output device means that the user 301 does not need to have to look at the communications device 101 to determine the output. Similarly, the use of the tactile outputs avoids the use for audio outputs that could interfere with the communication session and/or be picked up by the microphones 115 and transmitted to other participants in the communication session. In some examples the tactile output device could comprise a tactile audio display. The tactile audio display can be a display 109 that can be configured to provide a tactile output. For example, parts of the tactile display can be configured to vibrate and provide sensations that the user 301 can feel through their sense of touch. Other types of tactile output device could be used in other examples of the disclosure.

In the example of Fig. 6B a user 301 is making a user input to indicate that the identified emotional context is not correct. In this example the user input can comprise making a movement or other gesture with the communications device 101. In this example the input comprises the user 301 lifting the communications device 101 a short distance away from their ear and then moving it back again as indicated by the arrow 601. This motion can be detected by accelerometers within the communications device 101 or any other suitable means.

Other types of user input could be used to indicate that the identified emotional context is not correct. For example, the communications device 101 could be configured to detect the user tapping part of the communications device 101. The user 301 could make such tapping inputs while holding the communications device 101 to their ear.

In response to detecting the user input indicating that the identified emotional context is not correct the identified emotional context is rejected. In some examples the sensors 111 can continue to monitor the facial characteristics of the user 101 and can make another identification of the emotional context of the user 301. The process of enabling the user to confirm whether or not the correct emotional context has been identified can be repeated as many times as appropriate. In some examples a time-out period could begin if there are a number of incorrect identifications made within a threshold time period. The time-out period could be used to avoid annoying the user 301.

In some examples the communications device 101 can be configured to enable a user 301 to make a user input indicative that the identified emotional context is correct. For example, the user could make a different gesture with the communications device 101 compared to the one used to indicate that the identified emotional context is not correct. For example, the user 301 could move the communications device 101 in a different direction.

In some examples the user 301 could confirm that the identified emotional context is correct by not making any user input. For example, if the communications device 101 does not detect any input in response to the tactile output within a predetermined time period then it can be assumed that the correct emotional context has been identified.

If the correct emotional context has been identified then information indicative of the identified emotional context can be transmitted to the other participant device 305 and/or any other suitable function can be performed based on the emotional context.

In some examples of the disclosure the user inputs confirming whether or not the correct emotional context has been identified can be used to train or update the machine learning program.

The tactile output device could also be used to provide other outputs to the user 301. For instance, in examples where the user 301 is holding the communications device 101 to their ear then the user 301 is unable to view the display 109 and so is also unable to view images of the other participants in the communication session. In such examples the tactile output device could be used to provide an indication of the emotional context of the other participants in the call. For example, it could provide an indication as to whether the other participants are smiling or frowning. The information about the emotional context of the other participants could be obtained from analysis of the facial characteristics, analysis of images obtained of the other participants or by any other suitable means. The analysis could be performed by the user's device 101, by a device within the communications network, by one or more of the devices of the other participants or by any suitable device and/or combination of devices.

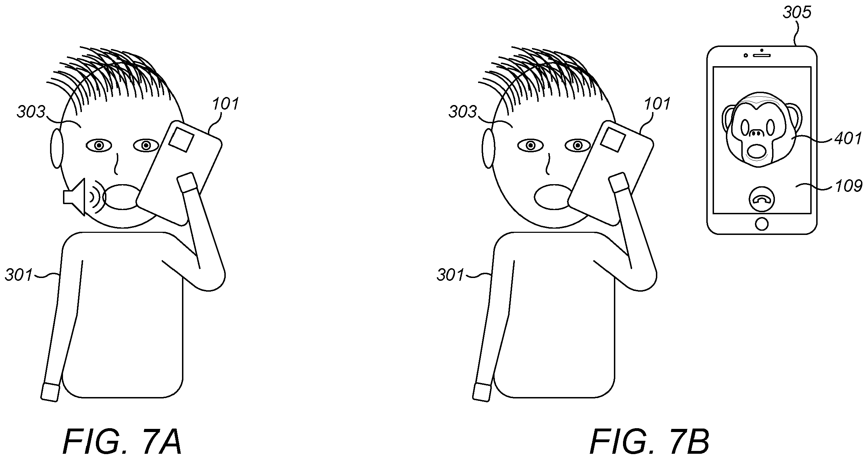

Figs. 7A and 7B show another example scenario for a communications device 101 in use. The communications device 101 could be a communications device 101 as shown in Fig. 1 or any other suitable type of device. In this example the communications device 101 is a handheld device such as a mobile phone. Other types of communications device 101 could be used in other examples.

In Fig. 7A the user is 301 is using the communications device 101 for a voice call or other similar communication session. The user 301 is holding the communications device 101 close to their ear for privacy purposes or for any other suitable reasons.

In the example of Fig. 7A the communications device 101 is configured so that the identification of the emotional context is performed when the user 301 is talking or making some other type of noise. In such examples the microphone signals from the microphones 115 can be monitored to detect when the user is talking 301 or making any other suitable noise.

In Fig. 7B noise or other suitable sounds are detected by the microphones 115. In this case the sensors 111 can be configured to monitor the facial characteristics of the user 301 and the communications device 101 can be configured to use the outputs of these sensors 111 to identify an emotional context of the user 301.

A visual representation 401 of the identified emotional context has been generated and is displayed on the display 109 of the other participant devices 305 in the communication session.

In the examples of Figs. 7A and 7B the processing requirements of the communications device 101 can be reduced because the facial characteristics of the user 301 only need to be monitored at certain times. In this example the times for monitoring the facial characteristics are the time periods during which the user 301 is talking or making other types of noise. Other time periods for monitoring the user's facial characteristics could be defined in examples of the disclosure.

In some examples other information in addition to the information form the sensors 111 can be used to help to identify the emotional context of the user 301. For example, the microphone signals could be monitored to obtain additional information. For instance, the microphone signals could be monitored to detect if the user 301 is laughing which could indicate that the emotional context of the user is happy.

In some examples the user 301 could make a user input indicating that they want other participants in the communication session to switch to a more private mode of operation. For example, the user 301 might want the other participants to switch to an earpiece mode or require that other participants hold their communication devices 101 to their ears. In such cases the user 301 could make an input or gesture that could be transmitted to the other participants in the communication session. Once all the participants are using the ear piece mode or holding their communications devices 101 to their ears then the monitoring of the facial characteristics to determine emotional contexts could be performed for the other participants in the communication session. The information about the emotional contexts could be provided to the user 301 via a tactile output or by any other suitable means.

In some examples the communications device 101 can be configured to enable a user to control whether or not they send information about their emotional context to the other participants in the communication session. For instance, when a user 301 moves their communications device 101 close to their ear they could also make a user input that indicates whether or not they want their facial characteristics to be monitored and/or their emotional context to be transmitted. This enables a user 301 to control whether or not they share their information.

In the examples shown in Figs. 3A to 7B the communications device 101 comprises a handheld device such as a mobile phone. Other types of communications device 101 could be used in other examples of the disclosure. For example, devices such as headsets or earbuds could comprise sensors that can detect changes in facial characteristics that could be detected even when it is not possible to obtain images of the user's face.

Fig. 8 schematically illustrates an apparatus 103 that can be used to implement examples of the disclosure. In this example the apparatus 103 comprises a controller 801. The controller 801 can be a chip or a chip-set. In some examples the controller can be provided within a communications device or any other suitable type of device.

In the example of Fig. 8 the implementation of the controller 801 can be as controller circuitry. In some examples the controller 801 can be implemented in hardware alone, have certain aspects in software including firmware alone or can be a combination of hardware and software (including firmware).

As illustrated in Fig. 8 the controller 801 can be implemented using instructions that enable hardware functionality, for example, by using executable instructions of a computer program 807 in a general-purpose or special-purpose processor 803 that can be stored on a computer readable storage medium (disk, memory etc.) to be executed by such a processor 803.

The processor 803 is configured to read from and write to the memory 805. The processor 803 can also comprise an output interface via which data and/or commands are output by the processor 803 and an input interface via which data and/or commands are input to the processor 803.

The memory 805 is configured to store a computer program 807 comprising computer program instructions (computer program code 809) that controls the operation of the controller 801 when loaded into the processor 803. The computer program instructions, of the computer program 807, provide the logic and routines that enables the controller 801 to perform the methods illustrated in Fig. 2 The processor 803 by reading the memory 805 is able to load and execute the computer program 807.

The apparatus 103 therefore comprises: at least one processor 803; and at least one memory 805 including computer program code 809, the at least one memory 805 storing instructions 809 that, when executed by the at least one processor 803, cause the apparatus 103 at least to perform:
determining that a communications device is positioned close to an ear of a user of the communication device during a communication session wherein the communications device comprises at least one display;
using sensors in or under the display of the communications device to monitor one or more facial characteristics of the user while the communication device is close to the user's ear; and
identifying an emotional context of the user based on the monitored one or more facial characteristics of the user.

As illustrated in Fig. 8 the computer program 807 can arrive at the controller 801 via any suitable delivery mechanism 811. The delivery mechanism 811 can be, for example, a machine readable medium, a computer-readable medium, a non-transitory computer-readable storage medium, a computer program product, a memory device, a record medium such as a Compact Disc Read-Only Memory (CD-ROM) or a Digital Versatile Disc (DVD) or a solid state memory, an article of manufacture that comprises or tangibly embodies the computer program 807. The delivery mechanism can be a signal configured to reliably transfer the computer program 807. The controller 801 can propagate or transmit the computer program 807 as a computer data signal. In some examples the computer program 807 can be transmitted to the controller 801 using a wireless protocol such as Bluetooth, Bluetooth Low Energy, Bluetooth Smart, 6LoWPan (IPᵥ6 over low power personal area networks) ZigBee, ANT+, near field communication (NFC), Radio frequency identification, wireless local area network (wireless LAN) or any other suitable protocol.

The computer program 807 comprises computer program instructions for causing an apparatus 103 to perform at least the following:
determining that a communications device is positioned close to an ear of a user of the communication device during a communication session wherein the communications device comprises at least one display;
using sensors in or under the display of the communications device to monitor one or more facial characteristics of the user while the communication device is close to the user's ear; and
identifying an emotional context of the user based on the monitored one or more facial characteristics of the user.

The computer program instructions can be comprised in a computer program 807, a non-transitory computer readable medium, a computer program product, a machine readable medium. In some but not necessarily all examples, the computer program instructions can be distributed over more than one computer program 807.

Although the memory 805 is illustrated as a single component/circuitry it can be implemented as one or more separate components/circuitry some or all of which can be integrated/removable and/or can provide permanent/semi-permanent/ dynamic/cached storage.

Although the processor 803 is illustrated as a single component/circuitry it can be implemented as one or more separate components/circuitry some or all of which can be integrated/removable. The processor 803 can be a single core or multi-core processor.

References to "computer-readable storage medium", "computer program product", "tangibly embodied computer program" etc. or a "controller", "computer", "processor" etc. should be understood to encompass not only computers having different architectures such as single /multi- processor architectures and sequential (Von Neumann)/parallel architectures but also specialized circuits such as field-programmable gate arrays (FPGA), application specific circuits (ASIC), signal processing devices and other processing circuitry. References to computer program, instructions, code etc. should be understood to encompass software for a programmable processor or firmware such as, for example, the programmable content of a hardware device whether instructions for a processor, or configuration settings for a fixed-function device, gate array or programmable logic device etc.

As used in this application, the term "circuitry" can refer to one or more or all of the following:
(a) hardware-only circuitry implementations (such as implementations in only analog and/or digital circuitry) and
(b) combinations of hardware circuits and software, such as (as applicable):
   (i) a combination of analog and/or digital hardware circuit(s) with software/firmware and
   (ii) any portions of hardware processor(s) with software (including digital signal processor(s)), software, and memory(ies) that work together to cause an apparatus, such as a mobile phone or server, to perform various functions and
(c) hardware circuit(s) and or processor(s), such as a microprocessor(s) or a portion of a microprocessor(s), that requires software (e.g. firmware) for operation, but the software can not be present when it is not needed for operation.

This definition of circuitry applies to all uses of this term in this application, including in any claims. As a further example, as used in this application, the term circuitry also covers an implementation of merely a hardware circuit or processor and its (or their) accompanying software and/or firmware. The term circuitry also covers, for example and if applicable to the particular claim element, a baseband integrated circuit for a mobile device or a similar integrated circuit in a server, a cellular network device, or other computing or network device.

The apparatus 103 as shown in Fig. 8 can be provided within any suitable device. In some examples the apparatus 103 can be provided within an electronic device such as a mobile telephone, a teleconferencing device, a camera, a computing device or any other suitable device. In some examples the apparatus is the communications device 101 or is an electronic device such as a mobile telephone, a teleconferencing device, a camera, a computing device or any other suitable device.

The blocks illustrated in Fig. 2 can represent steps in a method and/or sections of code in the computer program 807. The illustration of a particular order to the blocks does not necessarily imply that there is a required or preferred order for the blocks and the order and arrangement of the blocks can be varied. Furthermore, it can be possible for some blocks to be omitted.

The term 'comprise' is used in this document with an inclusive not an exclusive meaning. That is any reference to X comprising Y indicates that X may comprise only one Y or may comprise more than one Y. If it is intended to use 'comprise' with an exclusive meaning then it will be made clear in the context by referring to "comprising only one..." or by using "consisting".

In this description, reference has been made to various examples. The description of features or functions in relation to an example indicates that those features or functions are present in that example. The use of the term 'example' or 'for example' or 'can' or 'may' in the text denotes, whether explicitly stated or not, that such features or functions are present in at least the described example, whether described as an example or not, and that they can be, but are not necessarily, present in some of or all other examples. Thus 'example', 'for example', 'can' or 'may' refers to a particular instance in a class of examples. A property of the instance can be a property of only that instance or a property of the class or a property of a sub-class of the class that includes some but not all of the instances in the class. It is therefore implicitly disclosed that a feature described with reference to one example but not with reference to another example, can where possible be used in that other example as part of a working combination but does not necessarily have to be used in that other example.

Although examples have been described in the preceding paragraphs with reference to various examples, it should be appreciated that modifications to the examples given can be made without departing from the scope of the claims.

Features described in the preceding description may be used in combinations other than the combinations explicitly described above.

Although functions have been described with reference to certain features, those functions may be performable by other features whether described or not.

Although features have been described with reference to certain examples, those features may also be present in other examples whether described or not.

The term 'a' or 'the' is used in this document with an inclusive not an exclusive meaning. That is any reference to X comprising a/the Y indicates that X may comprise only one Y or may comprise more than one Y unless the context clearly indicates the contrary. If it is intended to use 'a' or 'the' with an exclusive meaning then it will be made clear in the context. In some circumstances the use of 'at least one' or 'one or more' may be used to emphasis an inclusive meaning but the absence of these terms should not be taken to infer any exclusive meaning.

The presence of a feature (or combination of features) in a claim is a reference to that feature or (combination of features) itself and also to features that achieve substantially the same technical effect (equivalent features). The equivalent features include, for example, features that are variants and achieve substantially the same result in substantially the same way. The equivalent features include, for example, features that perform substantially the same function, in substantially the same way to achieve substantially the same result.

In this description, reference has been made to various examples using adjectives or adjectival phrases to describe characteristics of the examples. Such a description of a characteristic in relation to an example indicates that the characteristic is present in some examples exactly as described and is present in other examples substantially as described.

Whilst endeavoring in the foregoing specification to draw attention to those features believed to be of importance it should be understood that the Applicant may seek protection via the claims in respect of any patentable feature or combination of features hereinbefore referred to and/or shown in the drawings whether or not emphasis has been placed thereon.

## Claims

1. An apparatus comprising means for:
determining that a communications device is positioned close to an ear of a user of the communication device during a communication session wherein the communications device comprises at least one display;
using sensors in or under the display of the communications device to monitor one or more facial characteristics of the user while the communication device is close to the user's ear; and
identifying an emotional context of the user based on the monitored one or more facial characteristics of the user.

2. An apparatus as claimed in claim 1 wherein the means are for enabling information indicative of the identified emotional context to be transmitted to one or more other participants in the communication session.

3. An apparatus as claimed in claim 2 wherein the means are for detecting a user input indicating whether or not the user of the communications device wants to transmit information indicative of the identified emotional context to one or more other participants in the communication session and determining whether or not to transmit the information indicative of the identified emotional context to the one or more other participants in the communication session based on the user input.

4. An apparatus as claimed in claim 2 wherein the information indicative of the identified emotional context is configured to enable a visual representation of the identified emotional context to be generated.

5. An apparatus as claimed in claim 4 wherein the communication session comprises a video call and the means are for using the visual representation of the identified emotional context to replace images from the imaging device of the communication device in the video call.

6. An apparatus as claimed in any preceding claim wherein the sensors in or under the display of the communications device comprise optical sensors.

7. An apparatus as claimed in any preceding claim wherein the one or more facial characteristics comprise one or more of: the position of one or more facial features, relative distances between two or more facial features, movement of one or more facial features, skin tone.

8. An apparatus as claimed in any preceding claim wherein the means are for controlling the communications device to provide an output to the user to indicate the identified emotional context.

9. An apparatus as claimed in claim 8 wherein the communications device comprises a tactile output device and the means are for controlling the tactile output device to provide the output indicating the identified emotional context.

10. An apparatus as claimed in any of claims 8 to 9 wherein the means are for detecting a user input indicating whether or not the identified emotional context is correct.

11. An apparatus as claimed in any preceding claim wherein the communications device comprises one or more microphones and the means are for analysing microphone output signals to determine when the user is speaking and controlling the monitoring of the one or more facial characteristics to monitor the one or more facial characteristics of the user while they are speaking.

12. An apparatus as claimed in any preceding claim wherein the means are for identifying the emotional context using a machine learning program.

13. A device comprising an apparatus as claimed in any preceding claim wherein the device is at least one of: a telephone, a camera, a computing device, a teleconferencing device, a virtual reality device, an augmented reality device.

14. A method comprising:
determining that a communications device is positioned close to an ear of a user of the communication device during a communication session wherein the communications device comprises at least one display;
using sensors in or under the display of the communications device to monitor one or more facial characteristics of the user while the communication device is held to the ear; and
identifying an emotional context of the user based on the monitored one or more facial characteristics of the user.

15. A computer program comprising computer program instructions that, when executed by processing circuitry, cause:
determining that a communications device is positioned close to an ear of a user of the communication device during a communication session wherein the communications device comprises at least one display;
using sensors in or under the display of the communications device to monitor one or more facial characteristics of the user while the communication device is held to the ear; and
identifying an emotional context of the user based on the monitored one or more facial characteristics of the user.
